# EUROPEAN PATENT APPLICATION

(11) **EP 2 589 412 A1**
(43) Date of publication of application: **08.05.2013**
(21) Application number: 11188036.5
(22) Date of filing: 07.11.2011
(51) Int. Cl.: A61N 5/10

(54) **Electronic brachytherapy source for use in /near MR scanners**

(71) Applicant: Koninklijke Philips Electronics N.V., 5621 BA Eindhoven (NL); Philips Intellectual Property & Standards GmbH, 20099 Hamburg (DE)
(72) Inventor: Amthor, Thomas, E., 5600 AE Eindhoven (NL); Weiss, Steffen, 5600 AE Eindhoven (NL); Overweg, Johan, 5600 AE Eindhoven (NL)
(74) Representative: Damen, Daniel Martijn

(57) **Abstract**

A miniature X-ray source (10) for high dose rate brachytherapy that can be operated in a wide range of operating directions (76) in the presence of a strong magnetic field (B), such as, for instance, the static magnetic field (B) of an MR scanner, with at least one anode (12) and at least one cathode (14), wherein in an operative state, an electric field (18) between the anode (12) and the cathode (14) is essentially spherically symmetric in at least a continuous solid angle of more than π/2 sr about a center (16) of the cathode (14); a brachytherapy system, comprising at least one said miniature X-ray source (10), and a method for generating a beam (82) of X-ray radiation inside an outer magnetic field (B) or an operative MR scanner with said miniature X-ray source (10).

## Description

### FIELD OF THE INVENTION

The invention pertains to a miniature X-ray source for use in a brachytherapy system, in particular an image-guided brachytherapy system, and a method for generating a beam of X-ray radiation in a magnetic field, in particular in/near a magnetic field of an operative MR scanner.

### BACKGROUND OF THE INVENTION

In high dose rate (HDR) brachytherapy of tumors it is known to apply miniaturized X-ray sources, to be inserted into the tumor by brachytherapy catheters. These X-ray sources show a number of specific advantages over conventionally used radioactive isotopes, an easier adjustment of radiation emission strength and radiation dose, the option of a continuous adjustment of an applied photon energy and much less effort regarding logistics being among those.

It is desirable to combine the advantages of the miniaturized X-ray sources with image-guiding techniques, to allow for a physician to easily control a position and alignment of each of applied X-ray sources. The widely used MR scanners would provide a suitable imaging for that purpose.

X-ray sources make use of a sudden deceleration of electrons at an anode electrode (anode) after having gained energy from a potential difference between the anode and a cathode electrode (cathode) they had been emitted from. In the presence of a static magnetic field (B0 field) of an MR scanner, the electrons, while being accelerated from the cathode to the anode, may experience a Lorentz force that acts perpendicular to a direction of motion of the electrons.

As long as the motion direction of the electrons is parallel to the magnetic field, the Lorentz force is zero and the X-ray source will work correctly. As soon as an angle between the direction of the magnetic field and the direction of motion of the electrons becomes non-zero, the electrons will be deflected and will in general begin to follow a helical path, and intended trajectories of the electrons will be considerably affected so that the X-ray source might no longer work properly because the electrons will not hit the anode any more.

Aligning the X-ray sources parallel to the magnetic field is not a viable option in practice due to physiological motions and limited accuracy of applicator placement.

### SUMMARY OF THE INVENTION

It is therefore an object of the invention to provide a brachytherapy system with an improved miniature X-ray source that can be operated in a wide range of operating directions in the presence of a strong magnetic field, such as, for instance, the static magnetic field of an MR scanner.

The phrase "strong magnetic field", as used in this application, shall be understood particularly as a magnetic field strength in relation to an accelerating voltage that results in a radius of an electron helical trajectory which is smaller than a gap between a cathode and an anode of the X-ray source.

In one aspect of the present invention, the object is achieved by a miniature X-ray source with at least one anode and at least one cathode, wherein in an operative state, an electric field between the anode and the cathode is essentially spherically symmetric in at least a continuous solid angle of more than π/2 sr about a center of the cathode.

The phrase "center of the cathode", as used in this application, shall be understood particularly as a geometric center of all parts of the cathode that emit electrons in the operative state.

The phrase "essentially spherically symmetric", as used in this application, shall be understood particularly such that, for a majority of more than 80% of equally spaced locations between the anode and the cathode, the electric field at any of these locations does not comprise any component that is perpendicular to a radial direction related to the center of the cathode, and that is larger than 30%, preferably larger than 15%, of an absolute value of the electric field strength at the location.

The phrase "a continuous solid angle", as used in this application, shall be understood particularly such that the solid angle, in terms of common spherical coordinates, can be described after the well-known projection towards a center of a unit sphere by the double integral ∫ ∫ sinθ dθ dϕ, and the integration is to be carried out over continuous intervals of the variables θ and ϕ.

The invention is based on the concept that, when the miniature X-ray source is disposed in an outer magnetic field and has a spherically symmetric electric field between the anode and the cathode, there will always be a path from the cathode to the anode where lines of the electric field and lines of the magnetic field are in parallel, so that electrons that are being emitted by the cathode in a direction of the electric field in the operative state of the X-ray source are not affected by the outer magnetic field. The continuous solid angle of at least π/2 sr (steradian), which for example is represented by a complete octant of the unit sphere, provides for a potentially wide range of operation of the X-ray source in terms of relative direction between the X-ray source and the orientation of the magnetic field.

The fact that a direction of the electron emission coincides with the direction of the magnetic field (or is antiparallel thereto) can be exploited to omit a specific space from being radiated by the X-ray source.

In a case where the outer magnetic field is essentially homogenous in a vicinity of the X-ray source, in the operative state of the X-ray source electrons are being emitted by the cathode and accelerated towards the anode in directions that are mainly directed both parallel and anti-parallel with regard to a direction of the outer magnetic field.

In another aspect of the present invention, the continuous solid angle which the electric field is essentially spherically symmetric in, is larger than 2π sr about a center of the cathode. With the complete space about the center of the cathode being 4π sr, a region of 2π sr provides for range of operation that may be wide enough to allow for an emission of electrons from the cathode and a successive acceleration to the anode in almost every configuration of the X-ray source relative to the outer magnetic field.

In a further aspect of the invention, the anode essentially completely encompasses the cathode. The phrase "to essentially completely encompass", as used in this application, shall be understood particularly such that the anode covers a solid angle of at least 80%, preferably of at least 90% of the complete solid angle of 4π sr about the center of the cathode. This configuration may provide for a maximum range of operation of the X-ray source in the presence of a strong magnetic field.

In a preferred embodiment, the anode may completely enclose the cathode except for an opening in the anode that is provided to accept an electrical cable feedthrough for supplying an electrical potential to the cathode.

In another aspect of the invention, the anode of the X-ray source may comprise at least two anode elements that in the operative state of the X-ray source form the anode. One element of the at least two elements of the anode is electrically insulated from a balance of the anode, and the electrically insulated element of the anode covers a continuous solid angle of more than (4π / 3) sr about the center of the cathode. In this configuration, electric field lines between the anode and the cathode may deviate from a spherical symmetry along a line from an interface region between the insulated anode element and the balance of the anode, to the cathode. Nevertheless, in a major part of space between the cathode and the balance of the anode the electric field is spherically symmetric. As a result, electrons are emitted by the cathode and accelerated to the balance of the anode, so that X-ray radiation would emerge from one side of the X-ray source only.

In another aspect of the invention, the cathode comprises a heatable filament provided for emitting electrons, allowing for a simple emission of electrons from the cathode. The filament could either be directly heated by passing a current through it, or indirectly, by providing a heating cartridge to the filament.

A rise in temperature of the cathode and/or a provision of a cooling liquid in order to hold a cathode temperature below an acceptable limit during operation of the X-ray source could be omitted when the cathode comprises a tip provided for emitting electrons by a field emission process.

A more homogeneous emission of electrons from the cathode can be obtained by furnishing an outer surface of the cathode with a plurality of field emission tips, as a portion of the outer surface of the cathode that emits electrons in the operative state that will eventually hit the anode, will comprise several field emission tips of the plurality of field emission tips.

The plurality of field emission tips may essentially be evenly distributed over the outer surface of the cathode. The phrase "evenly distributed", as used in this application, shall be understood particularly such that a standard deviation of spacing of adjacent field emission tips is less than 30%, preferably less than 20% of a mean value of the adjacent field emission tips spacing. An even distribution of the field emission tips provides the advantage that an X-ray emission pattern of the X-ray source shows only slight variations when the outer magnetic field direction changes.

The X-ray source may further comprise a shielding unit provided for absorbing X-ray radiation generated by the X-ray source in the operative state. The shielding unit can provide a certain degree of protection of regions which are intended to be left out from X-ray radiation exposure.

Preferably, the shielding unit may comprise one or more plate-like elements made from metal, for instance from lead. In a region of electron accelerating voltages up to 50 kV, a material thickness of the plate-like elements of a few tenth of a millimeter would be sufficient for effective radiation protection.

The shielding unit may cover a total solid angle of at least π/2 sr about the center of the cathode, and can thus provide an effective protection of a large portion of space to one side of the X-ray source.

It is another object of the invention to provide an image-guided brachytherapy system that can be operated in a wide range of operating directions in the presence of a strong magnetic field. This is attained by combining an embodiment of the X-ray sources of the invention with images from an MR scanner for guiding a position and/or direction of the X-ray source of the brachytherapy system. The image-guided brachytherapy system allows for real-time control of the X-ray source position and orientation, so that applicator fixation devices may become unnecessary.

### BRIEF DESCRIPTION OF THE DRAWINGS

These and other aspects of the invention will be apparent from and elucidated with reference to the embodiments described hereinafter. Such embodiment does not necessarily represent the full scope of the invention, however, and reference is made therefore to the claims and herein for interpreting the scope of the invention.

In the drawings:
Fig. 1 shows a greatly simplified view of an electrode arrangement of a miniature X-ray source,
Fig. 2 illustrates electron trajectories in the miniature X-ray source of Fig. 1 in the presence of a magnetic field,
Fig. 3a illustrates an alternative embodiment of a miniature X-ray source in a side view (left) and in a top view (right),
Fig. 3b illustrates another embodiment of a miniature X-ray source in a side view,
Fig. 3c illustrates a further embodiment of a miniature X-ray source in a side view,
Fig. 4 depicts a potential application for treating a tumor with a brachytherapy system comprising a miniature X-ray source and at a plurality of dwell positions,
Fig. 5 is a schematic illustration of an X-ray radiation pattern from a decelerated electron, and
Fig. 6 is a schematic side view of a magnetic stray field of an MR scanner.

### DETAILED DESCRIPTION OF EMBODIMENTS

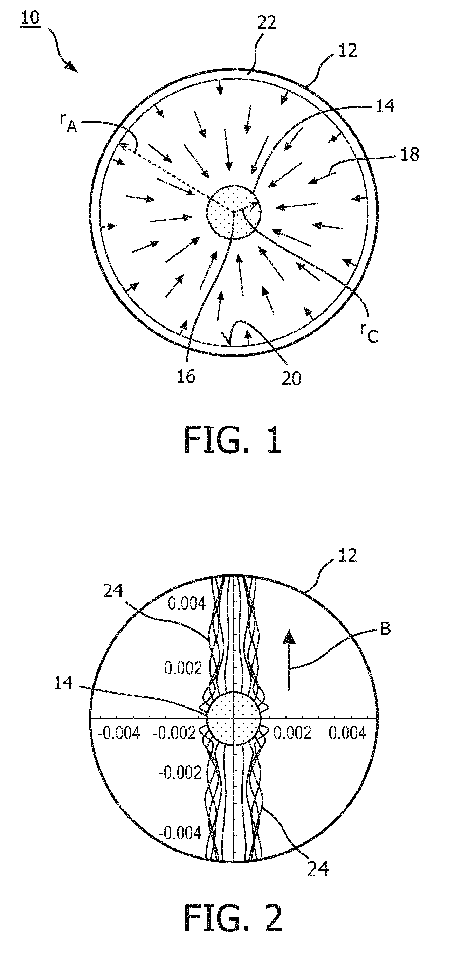

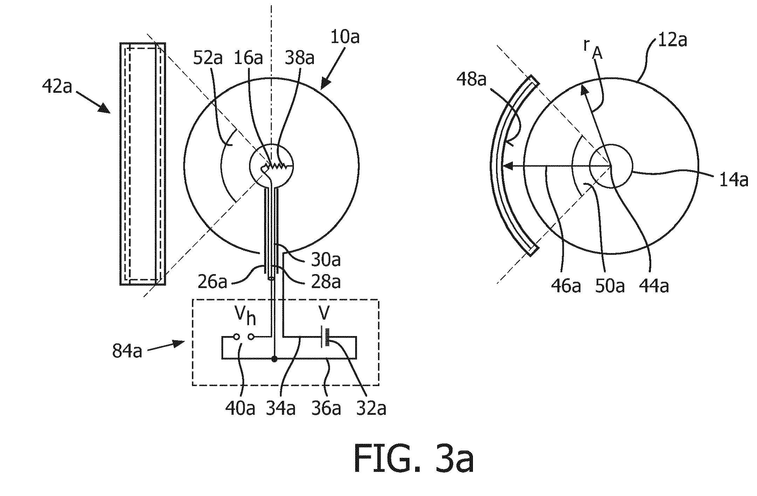

Fig. 1 shows a greatly simplified view of an electrode arrangement of a miniature X-ray source 10 with an anode 12 electrode (anode 12), essentially formed by a first sphere having a radius r_{A} of 5 mm and one cathode 14 electrode (cathode 14), essentially formed by a second sphere having a radius r_{C} of 1 mm. The spheres forming the anode 12 and cathode 14 are concentrically arranged. In an operative state, an accelerating voltage V (Fig. 3a-3c) is applied such that the anode 12 has a higher electrical potential than the cathode 14. A feedthrough (not shown) is provided in the anode 12 to take up an electric line to contact the cathode 14. Due to the spherical symmetry of the arrangement which is well-known from a spherical capacitor, an electric field 18 is created that is directed towards a center 16 of the cathode (which coincides with an origin of a spherical coordinate system) at all locations between the anode 12 and the cathode 14, and a magnitude of the electric field 18, indicated by a length of an arrow illustrating the electric field direction, varies as 1/r² for r_{C} ≤ r ≤ r_{A}.

Except for a slight disturbance in a vicinity of the feedthrough, the electric field 18 at almost every location in the space between the cathode 14 and the anode 12 essentially has a radial component only, and, therefore, the electric field 18 is essentially spherically symmetric in a solid angle of nearly 4π sr about the center 16 of the cathode 14.

The anode 12 is formed by a metal layer 20 designed as a metal foil or a metal coating on the inside of a heat-conducting substrate 22. In principle, it may alternatively be made from solid metal.

Electrons that are being emitted from the cathode 14 by a process to be explained subsequently will be accelerated in the electric field 18, with trajectories 24 that follow the electric field 18 lines, until they hit the anode 12, the sudden deceleration of the electrons generating X-ray radiation ("Bremsstrahlung") with a maximum photon energy that is equivalent to the energy gained by an electron in the full accelerating voltage V.

As illustrated in Fig. 2, this process is substantially altered in the presence of a magnetic field B. The electron trajectories 24 shown have been calculated for a homogeneous magnetic field B of a magnitude of 1.5 T and a direction in the plane of Fig. 2 that is indicated by the upward pointing arrow. The electrons that are emitted from the cathode 14 are strongly deflected and start spiraling around magnetic field lines that indicate the direction of the magnetic field B, the electrons being effectively accelerated in a direction parallel or anti-parallel to the magnetic field B by the electric field 18. Most of the emitted electrons will therefore reach the anode 12 to generate X-ray radiation. Because of the spherically symmetric electric field 18 (Fig. 1) between the cathode 14 and the anode 12, this holds for any direction of the magnetic field B.

Figs. 3a-3c show alternative embodiments of miniature X-ray sources. Components that are essentially like are assigned same reference signs or numerals. For differentiation, letters a, b or c are appended as an upper index to the reference numerals. As for function and features of the essentially like components, reference is made to a description of the embodiment shown in Fig. 1.

Fig. 3a shows a miniature X-ray source 10a comprising a cathode 14a and an anode 12a essentially formed by spheres, the cathode sphere and the anode sphere being arranged concentrically as described in Fig. 1, wherein the anode 12a essentially completely encompasses the cathode 14a except for an electrically insulated feedthrough 26a that is provided to take up two electric lines 28a, 30a and a tube provided for transporting a coolant agent to and from the cathode 14a for heat rejection (tube not shown).

A control unit 84a is provided for controlling an operation of the miniature X-ray source 10a, comprising a source 32a of an accelerating voltage V of 50 kV that is applied between the anode 14a and the cathode 12a by contacting the anode 14a directly with one terminal 34a of the source 32a and contacting one of the two electric lines 28a, 30a with the other terminal 36a of the source32a.

The cathode 14a comprises a heatable filament 38a with two contacting ends that is electrically contacted to the cathode 14a at the one contacting end and to one of the two electric lines 28a at the other contacting end. In an operative state of the X-ray source 10a, the filament 38a is directly heated by an applied heating voltage Vₕ from a second source 40a provided in the control unit 84b, to emit electrons by thermionic emission.

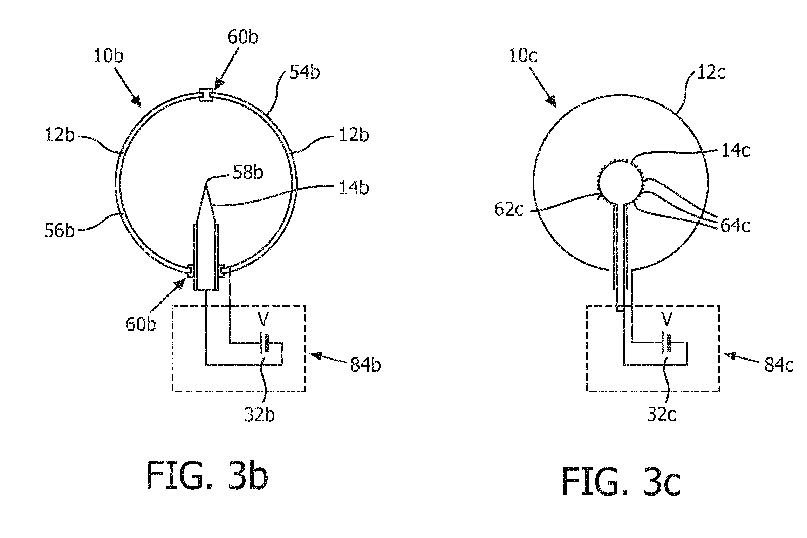

Furthermore, the miniature X-ray source 10a includes a shielding unit 42a that comprises a plastic-encased lead foil of 0.2 mm thickness and is provided for absorbing X-ray radiation generated by the X-ray source 10a in the operative state. The lead foil is shaped as a circular hollow cylinder that is cut in a direction parallel to a cylinder axis 44a to create a partial cylinder surface 48a (top view in Fig. 3c). A radius 46a of the cylinder is chosen slightly larger than the radius r_{A} of the anode sphere. The shielding unit 42a is arranged at a side of the anode sphere such that a convex side of the partial cylinder surface 48a faces an outer surface of the anode 12a. As can be taken from a side view in Fig. 3a, a height of the partial cylinder surface 48a is slightly larger than a diameter of the anode sphere. By this design, the shielding unit 42a covers a portion of the anode sphere that extends between an azimuth angle 50a of about 90° and also an elevation angle 52a of about 90°. In total, a solid angle of about 1.4 π/2 sr about the cathode center 16a is covered by the shielding unit 42a.

In Fig. 3b, the X-ray source 10b is furnished with a cathode 14b having a tip 58b that is provided for emitting electrons by field emission. The tip 58b has a radius of about 50 µm. In an operative state of the X-ray source 10b, the tip radius is small enough to create an electric field with a magnitude of about 10⁹ V/m, sufficient for field emission of electrons from the tip 58b. The X-ray source 10b has an anode 12b that consists of two anode elements 54b, 56b formed by two half-spheres that, in the operative state, form the anode 12b. A first anode element 54b of the two anode elements 54b, 56b is contacted to a source 32b providing an accelerating voltage V. The second anode element 56b of the two anode elements 54b, 56b is electrically insulated from a balance of the anode 12b by a nonconductive sealing unit 60b. Therefore, except for edge effects at an interface of the two anode elements 54b, 56b, the electric field 18b is spherically symmetric in almost one half-space, or a continuous solid angle of 2π sr. The electrically insulated anode element 56b, at which no X-ray radiation will be generated in an operative state of the X-ray source 10b, covers about a continuous solid angle of about 2π sr, that is to say, another half-space.

Fig. 3c shows the concentrical arrangement of the spheres forming the cathode 14c and the anode 12c, respectively, of the embodiment of Fig.3a. An outer surface 62c of the cathode 14c that is facing the anode 12c, however, is equipped with a plurality of field emission tips 64c that may be formed by carbon nanotubes and that are evenly distributed on the cathode outer surface 62c, resulting in an almost uniform field emission pattern for different orientations of a magnetic field B.

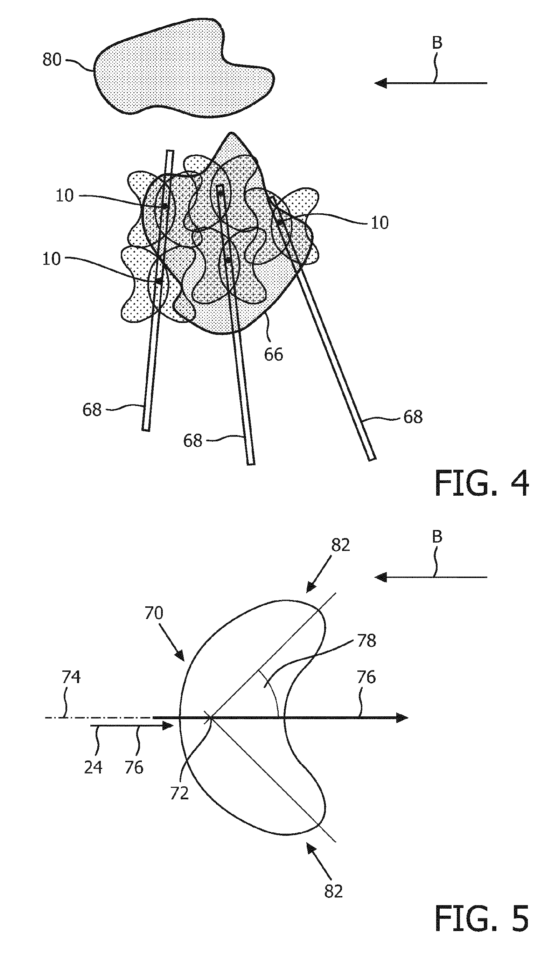

Fig. 4 shows a potential application for treating a tumor 66 with a brachytherapy system comprising a miniature X-ray source 10 of the type disclosed in Fig 3c that is successively placed at predefined dwell positions within a plurality of brachytherapy catheters 68 for a predefined operating time. Each of the dwell positions of the X-ray source 10 within the catheters 68 is indicated by a full dot. An external magnetic field B is present, the direction of which is illustrated in Fig. 4 by a leftward pointing arrow. The external magnetic field B is homogeneous in a pictured vicinity of the dwell positions of the X-ray source 10.

As described earlier, electrons are emitted from the cathodes 14 of the X-ray sources 10 in directions both parallel and anti-parallel to the external magnetic field B. In a cross-sectional side view, an intensity of an X-ray radiation beam 82 that is generated when an electron hits the anode 12 shows a double-lobe pattern 70, with the X-ray source 10 located near an apex 72 of the double-lobe pattern 70 and an axis 74 of symmetry of the double-lobe pattern 70 coinciding with a direction 76 of a trajectory 24 of the electron during deceleration (Fig. 5). Due to the rotational symmetry, in a three-dimensional viewing the radiation intensity pattern has a cone-like shape, with a recess at the cone base.

With an applied accelerating voltage V of 50 kV, the electrons have a velocity v in a mildly relativistic region with a value for β (= v/c, c: speed of light) of about 0.42, when hitting the anode 12. The emitted X-ray radiation intensity has a maximum intensity at an angle 78 of about 44°, the angle 78 being formed between the emitted radiation and the direction 76 of the trajectory 24 of the electron during deceleration, and is lower in the direction of the axis 74 of symmetry, accounting for the recess in the radiation pattern. For an accelerating voltage V of 100 kV, the value for β is about 0.55 and the maximum X-ray radiation is emitted at an angle 78 of 35°.

As shown, the X-ray radiation intensity is quite low in a direction that is perpendicular to the direction 76 of the electron trajectory 24 that extends parallel and anti-parallel to the external magnetic field B. In other words, the direction of the external magnetic field B and to some degree the accelerating voltage V are design parameters that an operator could make use of to define regions of low exposure and regions of high exposure to X-ray radiation from the X-ray source 10. The regions of high exposure to X-ray radiation may preferably be regions within the tumor 66, whereas regions of low exposure are, of course, most suited for placing objects like organs 80 that preferably should not be exposed to any radiation at all.

The external magnetic field B described in Figs. 2 and 4 may be a static magnetic field (B0 field) of an MR scanner being part of a brachytherapy system. Dimensions of a miniaturized X-ray source 10 are small in comparison to characteristic lengths of variations of the B0 field, so that the static magnetic field can be considered homogeneous in a vicinity of the X-ray source 10. Positions and/or directions of the X-ray source can be guided by MR images. Due to the properties of the X-ray sources described above, by knowledge of a direction of the magnetic field lines at the X-ray sources, regions of higher or lower exposure to X-ray radiation can be selected by the operator.

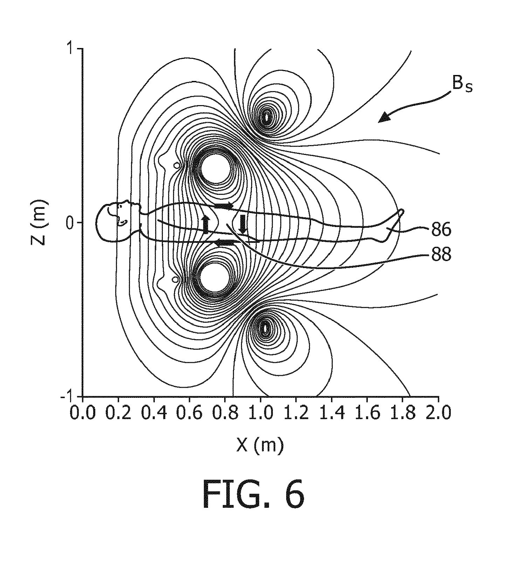

In an alternative configuration of the brachytherapy system which is shown in Fig. 6, also comprising an MR scanner, the external magnetic field described in Figs. 2 and 4 is represented by a magnetic stray field Bₛ of the MR scanner that is understood to continue to the left of Fig. 6 but is not shown therein. Four arrows in the center of Fig. 6 indicate directions of the magnetic stray field Bₛ in a center region 88 in which a selection of the X-ray radiation direction generated by an X-ray source (not shown) of a brachytherapy system placed therein can most conveniently be performed, as every potentially desired direction in the xz-plane that coincides with the drawing plane of Fig. 6 is readily available by shifting the brachytherapy system by a short distance. In Fig. 6, a contour 86 of a human body at an entrance position of the MR scanner is shown for comparison.

In another embodiment of a brachytherapy system, the external magnetic field described in Figs. 2 and 4 may alternatively be the magnetic field of a magnetic coil or an arrangement of several magnetic coils (like 3D-Helmholtz coils) or also from permanent magnets. In combination with a brachytherapy system comprising at least one miniaturized X-ray source, the operator could control a direction of emission of X-ray radiation by aligning the direction of the magnetic field lines in the vicinity of the X-ray source. In further combination with a simultaneous modulation of the intensity of the X-ray radiation, this device can allow for producing any desired pattern of radiation dose application in three dimensions.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### REFERENCE SYMBOL LIST

- 10: X-ray source
- 12: anode
- 14: cathode
- 16: cathode center
- 18: electric field
- 20: metal layer
- 22: heat-conducting substrate
- 24: trajectory
- 26: feedthrough
- 28: electric line
- 30: electric line
- 32: source
- 34: terminal
- 36: terminal
- 38: filament
- 40: source
- 42: shielding unit
- 44: cylinder axis
- 46: cylinder radius
- 48: cylinder surface
- 50: azimuth angle
- 52: elevation angle
- 54: anode element
- 56: anode element
- 58: tip
- 60: sealing unit
- 62: (cathode) surface
- 64: tip(s)
- 66: tumor
- 68: catheter
- 70: double-lobe pattern
- 72: apex
- 74: axis of symmetry
- 76: direction
- 78: angle
- 80: organ
- 82: radiation beam
- 84: control unit
- 86: contour
- 88: center region
- B: magnetic
- Bₛ: magnetic stray field
- r_{A}: radius
- r_{C}: radius
- V: accelerating voltage
- Vₕ: heating voltage

## Claims

1. A miniature X-ray source (10) with at least one anode (12) and at least one cathode (14), wherein in an operative state, an electric field (18) between the anode (12) and the cathode (14) is essentially spherically symmetric in at least a continuous solid angle of more than π/2 sr about a center (16) of the cathode (14).

2. The miniature X-ray source (10) as claimed in claim 1, wherein the continuous solid angle which the electric field (18) is essentially spherically symmetric in, is larger than 2π sr about a center (16) of the cathode (14).

3. The miniature X-ray source (10) as claimed in claim 1, wherein the anode (12) essentially completely encompasses the cathode (14).

4. The miniature X-ray source (10) as claimed in claim 1, wherein the anode (12) comprises at least two anode elements (54, 56) that in the operative state form the anode (12), and wherein one element (56) of the at least two elements (54, 56) of the anode (12) is electrically insulated from a balance of the anode (12), and the electrically insulated element (56) of the anode (12) covers a continuous solid angle of more than 4π / 3 sr about the center (16) of the cathode (14).

5. The miniature X-ray source (10) as claimed in claim 1, wherein the cathode (14) comprises a heatable filament (38) provided for emitting electrons.

6. The miniature X-ray source (10) as claimed in claim1, wherein the cathode (14) comprises a tip (58; 64) provided for emitting electrons by field emission.

7. The miniature X-ray source (10) as claimed in claim 1, further comprising a shielding unit (42) provided for absorbing X-ray radiation generated by the X-ray source (10) in the operative state.

8. The miniature X-ray source (10) as claimed in claim 7, wherein a total solid angle of at least π/2 sr about the center (16) of the cathode (14) is covered by the shielding unit (42).

9. The miniature X-ray source (10) as claimed in claim 1, wherein an outer surface (62) of the cathode (14) is equipped with a plurality of field emission tips (64).

10. The miniature X-ray source (10) as claimed in claim 9, wherein the plurality of field emission tips (64) is essentially evenly distributed over the outer surface (62) of the cathode (14).

11. A brachytherapy system, comprising
- at least one miniature X-ray source (10) as claimed in one of the preceding claims, and
- at least one control unit (84) provided for controlling an operation of the at least one miniature X-ray source (10).

12. The brachytherapy system as claimed in claim 11, further comprising an MR scanner for guiding a position and/or direction of the X-ray source (10) of the brachytherapy system by at least one MR image.

13. A method for generating a beam of X-ray radiation with at least one miniature X-ray source (10) in a magnetic field (B) inside of the MR scanner in an operative state as claimed in claim 12, comprising the following steps:
- guiding the miniature X-ray source (10) to a desired position inside the operative MR scanner by at least one image created by the MR scanner in the operative state
- aligning the X-ray source (10) to any desired direction in space

14. A method for generating a beam (82) of X-ray radiation with at least one miniature X-ray source (10) as claimed in claim 1, the beam (82) of X-ray radiation to be propagating in a desired direction, the method comprising the following step:
- creating a magnetic field (B) in a vicinity around the miniature X-ray source (10), wherein the magnetic field (B) is essentially directed in the desired direction of propagation of the X-ray beam (82)

15. The method for generating a beam (82) of X-ray radiation as claimed in claim 14, wherein the magnetic field (B) in the vicinity around the miniature X-ray source (10) is formed by a magnetic stray field (Bₛ) of an MR scanner in an operative state.
